(19) Europäisches Patentamt
European Patent Office
Office européen des brevets

(11) **EP 2 044 414 B1**

(12) **EUROPÄISCHE PATENTSCHRIFT**

(45) Veröffentlichungstag und Bekanntmachung des Hinweises auf die Patenterteilung:
**14.09.2011   Patentblatt 2011/37**

(21) Anmeldenummer: **07765199.0**

(22) Anmeldetag: **13.07.2007**

(51) Int Cl.:
***G01N 11/10*** *(2006.01)*

(86) Internationale Anmeldenummer:
**PCT/EP2007/006257**

(87) Internationale Veröffentlichungsnummer:
**WO 2008/011996 (31.01.2008 Gazette 2008/05)**

(54) **VERFAHREN UND VORRICHTUNG ZUM PERIODISCHEN MESSEN DER FLIESSGRENZE VON DISPERSIONEN SOWIE DEREN VERWENDUNG**

METHOD AND APPARATUS FOR PERIODICALLY MEASURING THE YIELD POINT OF DISPERSIONS, AND USE THEREOF

PROCÉDÉ ET DISPOSITIF DE MESURE PÉRIODIQUE DE LA LIMITE D'ÉCOULEMENT DE DISPERSIONS ET LEUR UTILISATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **25.07.2006   DE 102006034346**

(43) Veröffentlichungstag der Anmeldung:
**08.04.2009   Patentblatt 2009/15**

(73) Patentinhaber: **Fraunhofer-Gesellschaft zur Förderung der angewandten Forschung e.V.
80686 München (DE)**

(72) Erfinder:
• **ZIEGLEDER, Gottfried
81245 München (DE)**
• **BEAURY, Alexander
83224 Grassau (DE)**

(56) Entgegenhaltungen:
**DE-A1- 3 611 867     DE-C1- 19 515 250
US-A- 2 747 399**

EP 2 044 414 B1

**Beschreibung**

Die vorliegende Erfindung beschreibt ein Verfahren und eine Vorrichtung zum Messen der Fließgrenze von fließfähigen Stoffen, wobei ein Messkörper periodisch von dem zu messenden fließfähigen Stoff benetzt wird. Desweiteren wird eine Verwendung der Vorrichtung angegeben.

Hochviskose Dispersionen zeigen Nicht-Newton'sches Fließverhalten, d.h. sie weisen neben einer gewissen Viskosität der bewegten Masse noch eine Fließgrenze der Masse auf. Diese Fließgrenze, zu verstehen als innere Reibung der dispergierten Partikel in dichter Packung, muss zunächst überwunden werden, um die Masse aus dem Ruhezustand in Bewegung zu versetzen und in Bewegung zu halten.

Das Fließverhalten von Schokoladenmassen ist untersucht und wird nach offiziellen Methoden (OICCC) in der Schmelze bei 40 °C gemessen. Dabei wird nach Viskosität und Fließgrenze unterschieden. Zur Messung dienen heute Rheometer im Bereich eines Schergefälles zwischen ca. 0 und 200 s$^{-1}$. Beim kontinuierlichen Durchlaufen eines steigenden oder fallenden Schergefälles in diesem Bereich wird eine sog. Fließkurve registriert, die die Scherkraft über der Schergeschwindigkeit darstellt.

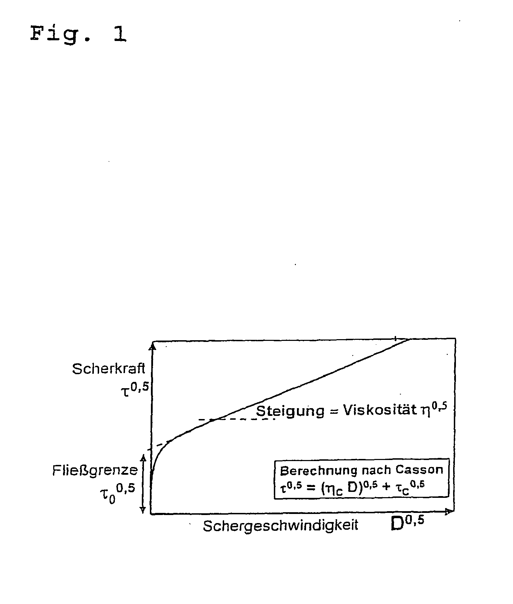

Die Messung und Auswertung von Fließkurven geschmolzener Schokoladen bei 40 °C ist umfangreich in der Literatur beschrieben (beispielsweise J. Chevalley. Die Fließeigenschaften von Schokolade. In: S. T. Beckett: Moderne Schokoladentechnologie. Behrs Verlag 1990, S. 211-231; H. D. Tscheuschner, Schokolade, Süßwaren. In: D. Weipert, H.D. Tscheuschner, E. Windhab. Rheologie der Lebensmittel. Behrs Verlag 1993, S. 431-470; H. D. Tscheuschner. Rheologische Eigenschaften von Schokoladenmasse und deren prozessrelevanten Bedeutung. Zucker- u. Süßwarenwirtschaft ZSW 1993, 136-147; Windhab E., Rolfes L. Messung des Fließverhaltens von Schokoladenmassen ZSW 1991, 401). Die Auswertung von Fließkurven und näherungsweise Berechnung der Fließgrenze erfolgte bisher nach Casson (Untersuchungsmethoden des Office International du Cacao et du Chocolat. Methode Blatt 10-D/1973. Viskosität von Schokolade - Bestimmung der Casson-Fließgrenze und der Casson Viskosität (Verlag Glättli, Postfach, CH-6934 Bioggio (Schweiz)) und in neuer Version nach Windhab (International Confectionery Association, "Viscosity of cocoa and chocolate products", Analytical Method 46, S. 1-17, ICA, rue Defacqz 1, 1000 Brussels (Belgium), Bestimmung der dynamischen Viskosität). Mögliche andere Näherungsverfahren für die Fließgrenze wurden an anderer Stelle diskutiert (Windhab E. Structure-Rheology Relationships in chocolate processing. In: G. R. Ziegler: Confectionery Science. Proceedings Penn State University 1997, Dep Food Science, 104-126). Das Prinzip einer Fließgrenze allgemein wird in zwei weiteren Publikationen (Coussot P, Boyer S. Determination of yield stress fluid behaviour from inclined plane test. Rheol. Acta 34: 534-543 (1995); Cousset P. Avalanche behaviour in yield stress fluids. Physical Review Letters, Vol. 88, No. 17, 175501-175504, (2002)) beschrieben und ist in Fig. 1 verdeutlicht.

Ein einfacher Haftmengentest für Schokoladenmassen und Kuvertüren ist aus der Literatur bekannt (J. Kleinert: Handbuch der Kakaoverarbeitung und Schokoladeherstellung, Behr's Verlag, 1997, S. 402ff.). Allerdings lässt sich das dargestellte Verfahren nur manuell ausführen und eignet sich nicht für eine quasi-kontinuierliche Überwachung von Schokoladenschmelzen. Ein weiterer Nachteil des Verfahrens ist, dass die dortige Verfahrensführung sehr umständlich gestaltet ist, da die verwendeten Testkörper nach der Benetzung mit der Schokoladenmasse zunächst gekühlt werden müssen um die anhaftende Resthaftmenge zu bestimmen. Außerdem ist eine aufwendige Mehrfachbestimmung vorgeschrieben.

Das Prinzip der schiefen Ebene wird in verschiedenen Publikationen beschrieben (Coussot P, Boyer S. Determination of yield stress fluid behaviour from inclined plane test. Rheol. Acta 34: 534-543 (1995); Cousset P. Avalanche behaviour in yield stress fluids. Physical Review Letters, Vol. 88, No. 17, 175501-175504, (2002); W. Bartusch. Der Einfluss von Rüttelstößen auf die Fließeigenschaften von Schokoladenmassen. Fette Seifen Anstrichmittel 63 (1961) 721-729; A. Beaury. Semesterarbeit Fraunhofer IVV/FH Weihenstephan, in Vorbereitung, 2006). Die Fließgrenze kann anhand des Abfließverhaltens einer Schokoladenmasse von einer definierten Oberfläche gemessen und berechnet werden. Die Prüffläche kann dabei bei gewissem Winkel $\alpha$ schräg geneigt sein ("schiefe Ebene") oder senkrecht stehen ($\alpha = 90°$). Entweder wird der Winkel $\alpha_1$ erfasst, bei dem die Masse gerade zu fließen beginnt, oder die Masse bestimmt, die nach Abschluss des Fließvorgangs bei gegebenem Winkel $\alpha > \alpha_1$ auf der Fläche verbleibt. Das Messverfahren mit einer senkrechten Prüfplatte und Erfassung der Restmasse wurde erstmals von Bartusch in statistischen Labortests bei 40 °C genutzt, um die Wirkung von auf Schokoladenschmelzen zu untersuchen. Einfache Auslauftrichter, die in einer weiteren Publikation (S. T. Beckett. The Science of chocolate. RSC Paperbacks. ISBN 0-85404-600-3, S. 112) beschrieben sind, oder das Bostwick Consistometer (Bostwick Consistometer. CSC Scientific Company Inc., 2003) funktionieren ebenfalls nach einem Ablaufprinzip auf einer schiefen Ebene, messen allerdings die Geschwindigkeit des Ablaufens und erfassen damit die Viskosität und nicht die Fließgrenze.

Für eine benetzte schräge Ebene oder geneigte Oberfläche mit Neigungswinkel $\alpha$ gilt folgende Gleichung zur Berechnung der Fließgrenze r

$$\tau = \frac{m \cdot g \cdot \sin \alpha}{A}$$

mit:

A = benetzte Fläche
$\alpha$ = Neigungswinkel
m = Masse der Resthaftmenge
g = Erdbeschleunigung 9,81 m/s$^2$

Für senkrechte Flächen der Messkörper ($\alpha$ = 90 °C und somit sin $\alpha$ = 1) gilt entsprechend:

$$\tau = \frac{m \cdot g}{A}$$

Aufgabe vorliegender Erfindung ist es, ein einfaches Verfahren und eine Vorrichtung bereitzustellen, mit der eine quasi-kontinuierliche Bestimmung der Fließgrenze von Dispersionen auf einfache und reproduzierbare Weise erfolgen kann. Weitere Aufgabe der Erfindung ist es, ein möglichst breites Spektrum an fließfähigen Stoffen durch das Messverfahren abzudecken.

Diese Aufgabe wird bezüglich des Verfahrens durch den Patentanspruch 1, bezüglich der Messvorrichtung durch den Patentanspruch 16 sowie bezüglich der Verwendung durch den Patentanspruch 24 gelöst. Die abhängigen Ansprüche stellen vorteilhafte Weiterbildungen dar.

Erfindungsgemäß wird ein Verfahren zum Messen der Fließgrenze von fließfähigen Stoffen in einem Strom des jeweiligen fließfähigen Stoffes bereitgestellt, wobei ein Messkörper iterativ von dem zu messenden fließfähigen Stoff bei gegebener, konstanter Temperatur benetzt wird, man anschließend den fließfähigen Stoff partiell für eine vorgegebene Ablaufzeit, die so bemessen ist, dass der Messkörper näherungsweise Gewichtskonstanz erreicht, ablaufen lässt und über die verbleibende Resthaftmenge am Messkörper die Fließgrenze bestimmt wird.

Das Auswahlkriterium für die Ablaufzeit ist dabei, dass die Ablaufzeit so bemessen ist, dass der Messkörper näherungsweise Gewichtskonstanz erreicht. Erfindungsgemäß wird darunter verstanden, dass die relative Änderung des Gewichtes des Messkörpers dabei < 1 % pro Sekunde beträgt. In der Regel hängt die Ablaufzeit von der Viskosität des zu messenden fließfähigen Stoffes ab. Je viskoser der zu messende Stoff ist, desto länger wird auch die Ablaufzeit.

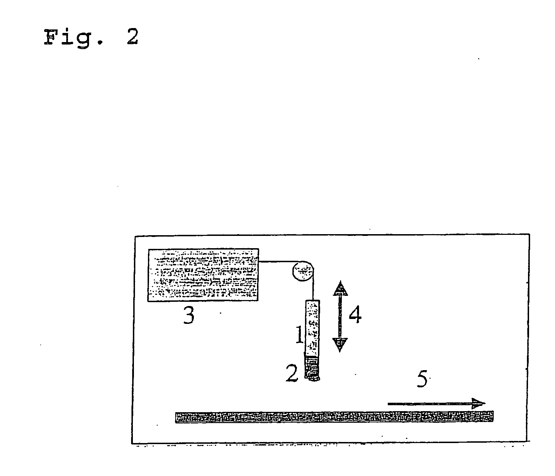

Zum einen kann das Messverfahren so erfolgen, dass der Messkörper in den fließfähigen Stoff eingetaucht und wieder herausgezogen wird. Dieses Prinzip ist in Fig. 2 dargestellt.

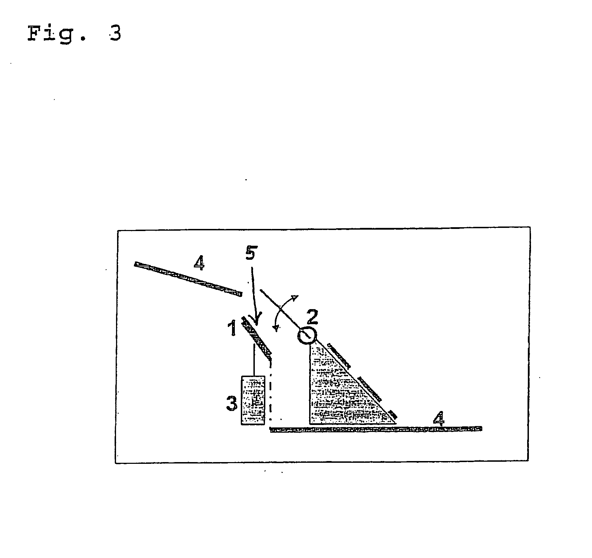

Als alternative Ausführungsform wird der Messkörper dabei von dem fließfähigen Stoff überspült. Diese Ausführungsform wird durch Fig. 3 wiedergegeben.

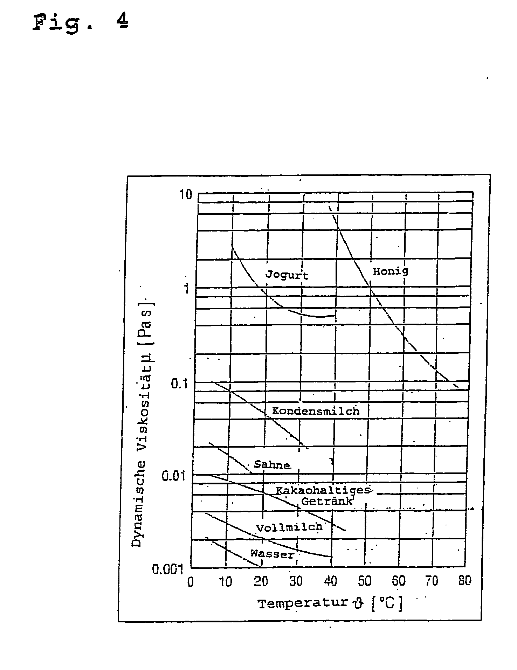

In jedem Fall wird die Zeit des Eintauchens bzw. Überspülens des Messkörpers in Abhängigkeit von der Viskosität des zu messenden fließfähigen Stoffes gewählt und ist so gemessen, dass eine vollständige Benetzung des Messkörpers und/oder ein vollständiger Austausch des fließfähigen Stoffes gewährleistet sind. Dabei sind je nach Fließverhalten des zu messenden Stoffes unterschiedliche Zeiten erforderlich. Diese bewegen sich bei niederviskosen Stoffen mit einer dynamischen Viskosität von beispielsweise 1 mPas bis 1 Pas im Bereich von 10 s bis 30 s, bei höherviskosen Stoffen mit einer Viskosität im Bereich von beispielsweise 1 Pas bis 100 Pas in der Größenordnung von 30 s bis 300 s, ohne die Eintauchzeiten auf die angegebenen Zeiten einschränken zu wollen. Ein wichtiger Vorteil des Verfahrens ist dabei, dass durch die gewählten Eintauchzeiten gewährleistet ist, dass die anhaftende Resthaftmenge vollständig ausgetauscht wird und der Messkörper auf dieselbe Temperatur wie der zu untersuchende fließfähige Stoff temperiert wird. Tabelle 1 gibt beispielhaft eine Übersicht über die dynamischen Viskositäten einer Auswahl an Dispersionen, deren Fließgrenze mit vorliegendem Verfahren bestimmt werden kann. Fig. 4 zeigt die Abhängigkeit der Viskositäten verschiedener Stoffe in Abhängigkeit von der Temperatur. Anhand dieses Sachverhaltes wird klar, dass es essentiell ist, den Messkörper auf die gleiche Temperatur wie den zu messenden Stoff einzustellen, um dessen rheologische Eigenschaften möglichst konstant zu halten.

Nach dem partiellen Ablaufen des fließfähigen Stoffes erfolgt eine Bestimmung des Gewichts und/oder des Volumens der dem Messkörper anhaftenden Resthaftmenge des fließfähigen Stoffes.

Die Bestimmung des Gewichts und/oder des Volumens der Resthaftmenge erfolgt dabei vorteilhafterweise über Sensoren ausgewählt aus der Gruppe bestehend aus Waage, Kraftaufnehmer, Schwingungssensor, optischen Sensoren, elektromagnetischen Sensoren, piezoelektrischen Sensoren und/oder Druckaufnehmern.

In einer weiteren vorteilhaften Ausführungsform des Verfahrens werden vertikale und/oder geneigte Flächen des Messkörpers benetzt.

Vorteilhafterweise wird das Verfahren so ausgeführt, dass die anhaftende Restmenge bei der Messung die annähernd gleichen rheologischen Eigenschaften wie der fließfähige Stoff, dem sie entnommen wurde, aufweist. Hierunter sind beispielsweise die dynamische Viskosität, Fließgrenze und alle physikalischen Eigenschaften, die von der Temperatur abhängen gemeint. Durch das iterative, periodische Eintauchen bzw. Überspülen des Messkörpers mit dem zu messenden fließfähigen Stoff wird diese Bedingung gewährleistet, da ein stetiger Austausch des fließfähigen Stoffes erfolgt. Dadurch ist auch gewährleistet, dass der Messkörper stets die gleiche Temperatur wie der zu messende Stoff aufweist und der Messkörper somit nicht zur Verfälschung der rheologischen Eigenschaften des zu untersuchenden Stoffes beiträgt.

Zur weiteren Absicherung dieser Bedingung dient auch die weitere alternative Ausführungsform, dass zwischen zwei Messzyklen der Messkörper in dem fließfähigen Stoff temperiert und/oder gespült wird.

Bevorzugt werden die ermittelten Messdaten elektronisch weiterverarbeitet und/oder gespeichert.

Erfindungsgemäß können die zu messenden Dispersionen ausgewählt sein aus der Gruppe bestehend aus viskosen Massen im Lebensmittel- oder Pharmabereich, Kosmetika, Dispersionsfarben und/oder keramischen Massen.

Dispersionen aus dem Lebensmittel-, Kosmetik- und/oder Pharmabereich können beispielsweise ausgewählt sein aus der Gruppe bestehend aus Schokoladenschmelzen, Jogurt, Ketchup, Brotaufstrich, Marmelade und/oder Honig bzw. Cremes, Salben, Lotionen, Lippenstift etc.

Insbesondere bei Schokoladenmassen eignet sich das Verfahren überraschenderweise besonders gut, um auf einfache Art und Weise eine Bestimmung des Temperiergrades, d.h. des Kristallisationsgrades von Schokoladenmassen zu bestimmen.

In einer weiteren bevorzugten Ausführungsform des Verfahrens wird die Messung der Fließgrenze mit einer Messung der Temperatur des fließfähigen Stoffes kombiniert, d.h. erfindungsgemäß werden die Messparameter - also Eintauch- und Ablaufzeit - in Abhängigkeit der Temperatur des fließfähigen Stoffes gewählt.

Insbesondere, wenn alle Faktoren, die die Rheologie des zu messenden Stoffes maßgeblich bestimmen, berücksichtigt werden, wird eine möglichst genaue, reproduzierbare und zuverlässige Messung der Fließgrenze ermöglicht. Insbesondere die Temperatur hat hohen Einfluss auf die dynamische Viskosität und somit die Rheologie des zu messenden Stoffes. Die Abhängigkeit der dynamischen Viskosität ausgewählter Stoffe ist in Fig. 4 widergegeben.

Erfindungsgemäß wird ebenso eine Messvorrichtung zum periodischen Messen der Fließgrenze von fließfähigen Stoffen bereitgestellt, mit einem Messkörper mit definierter Oberfläche, der mit einer funktionellen Einheit zur Bestimmung der am Messkörper nach der Abtropfzeit verbleibenden, anhaftenden Resthaftmenge des fließfähigen in Verbindung steht und ein Mittel zur Gewährleistung der periodischen Benetzung des Messkörpers mit der Dispersion.

In einer Ausführungsform kann das zur Gewährleistung der periodischen Messung des Messkörpers enthaltene Mittel eine Hebevorrichtung zur Absenkung und Anheben des Messkörpers in bzw. aus dem fließfähigen Stoff sein.

In einer alternativen Ausführungsform ist das Mittel eine Vorrichtung zum Abzweigen einer Teilmenge des fließfähigen Stoffes und zur Umleitung der Teilmenge auf den Messkörper. Dies können beispielsweise ein mechanischer Schieber, eine Klappe und/oder ein Ventil sein.

Prinzipiell kann ein Messkörper jede beliebige Form haben, z.B. die Form eines Stabes, einer Platte, eines Trichters, eines Kegels und/oder einer Kugel.

Vorzugsweise sollte der Messkörper vertikale und/oder geneigte Flächen, die von dem fließfähigen Stoff benetzt werden, aufweisen.

Die funktionelle Einheit, mit der die Restmenge des anhaftenden fließfähigen Stoffes am Messkörper bestimmt wird, ist vorteilhafterweise ausgewählt aus der Gruppe bestehend aus einer Waage, Kraftaufnehmer, Schwingungssensor, optischen Sensoren, elektromagnetischen Sensoren, piezoelektrischen Sensoren und/oder Druckaufnehmern.

Vorteilhafterweise ist weiterhin eine elektronische Auswertungseinheit zur Weiterverarbeitung und/oder Speicherung der Messdaten, vorzugsweise ein Computer vorgesehen.

Bevorzugt eignet sich das Verfahren zur kontinuierlichen Überwachung von fließfähigen Stoffen in einer Produktionsanlage.

Ebenso eignet sich vorliegendes Verfahren zur Bestimmung des Temperiergrades, d.h. des Kristallisationsgrades von Schokoladenmassen.

Anhand folgender Figuren soll vorliegende Erfindung näher erläutert werden, ohne dass die Erfindung auf die dort dargestellten Ausführungsformen eingeschränkt werden soll.

Fig. 2 beschreibt die Ausführungsform der Erfindung, bei der der Messkörper in den zu messenden fließfähigen Stoff durch Anheben und Absenken eingetaucht wird.

Fig. 3 beschreibt die Ausführungsform vorliegender Erfindung, bei der ein Teil eines fließenden Stromes des zu messenden fließfähigen Stoffes durch Abzweigung auf den Messkörper umgeleitet wird.

Fig. 4 verdeutlicht die Abhängigkeit der dynamischen Viskosität ausgewählter Stoffe von der Temperatur.

Fig. 5 zeigt anhand der Beispiele Bitterschokolade und Kakaobutter die Auswirkung des Gehalts an Fettkristallen auf die Fließgrenze.

Ein Messkörper 1 mit definierter Oberfläche wird periodisch in eine fließende Masse 5 (z.B. Schokoladenmasse) eingetaucht (Fig. 2) oder von dieser Masse 4 periodisch überspült (Fig. 3). Nach gewisser Kontaktzeit des Messkörpers mit der Masse 5 bzw. 4 wird der Messkörper 1 aus dem Massestrom 5 entnommen bzw. der Massezufluss 4 unterbrochen. Dies geschieht in der in Fig. 2 dargestellten Ausführungsform durch Herausziehen des Messkörpers 1 aus dem Massestrom 5, in der in Fig. 3 dargestellten Ausführungsform durch Betätigen eines Schiebers 2, durch den der Massestrom 4 umgelenkt wird. Nachdem frei fließende Masse vom Messkörper 1 abgelaufen ist, wird die noch anhaftende Restmasse 2 (Fig. 2) bzw. 5 (Fig. 3) gemessen. Die Messung erfolgt durch Bestimmung des Gewichtes oder Volumens der auf dem Messkörper 1 verbleibenden Masseschicht 2 (Fig. 2) bzw. 5 (Fig. 3), wobei je nach Lösungsweg eine Waage, ein Magnet, ein Kraftaufnehmer oder ein Schwingungssensor etc. zum Einsatz kommen kann. Aus der anhaftenden Restmasse 2 (Fig. 2) bzw. 5 (Fig. 3) lässt sich nach bekannten Gesetzen der schiefen Ebene die Fließgrenze berechnen; diese Daten werden elektronisch registriert. Die Messung erfolgt so schnell, dass die Masse während der Messung unverändert bleibt, d.h. dass beispielsweise Schokoladenmasse sich nicht durch Kristallisation verfestigen kann.

Durch das wiederholte Eintauchen bzw. Überspülen ist gewährleistet, dass die aus der vorhergehenden Messung anhaftende Restmasse 2 (Fig. 2) bzw. 5 (Fig. 3) wieder abgespült und eine neue Messung möglich wird. Gleichzeitig dient das wiederholte Eintauchen und Herausziehen 4 (Fig. 2) bzw. Überspülen durch Betätigen eines Zwei-Wege-Ventils für periodisches Abzweigen eines Teilstroms 2 (Fig. 3) der dauerhaften Temperierung des Messkörpers 1 auf die aktuelle Temperatur der Masse. Periodisches Eintauchen 4 wird durch einen Hebemechanismus 3 (Fig. 2) möglich, periodisches Überspülen beispielsweise durch das Abzweigen eines Teilstroms durch regelmäßiges Öffnen und Schließen eines Ventils 2 (Fig. 3). Ein Vorteil dieses Verfahrens ist, dass die eigentliche Bestimmung der Fließgrenze nicht durch den Fließvorgang der Masse gestört wird.

Bei temperierten Schokoladenmassen kann aus der gemessenen Fließgrenze auf den Temperiergrad (d.h. die Vorkristallisation) geschlossen werden, da sich diese stark auf die Fließgrenze auswirkt. Diese Messung des Temperiergrads (= Anteil an kristallisiertem Fett in der vorkristallisierten, noch fließfähigen Masse) ist von besonderer Bedeutung, da Temperiergrad und Fließverhalten von temperierten Massen nach bisherigen Methoden nur schwer kontinuierlich zu erfassen sind und deshalb in der industriellen Praxis nur selten gemessen werden.

**Vergleichsbeispiel**

Vorkristallisation von Schokoladen

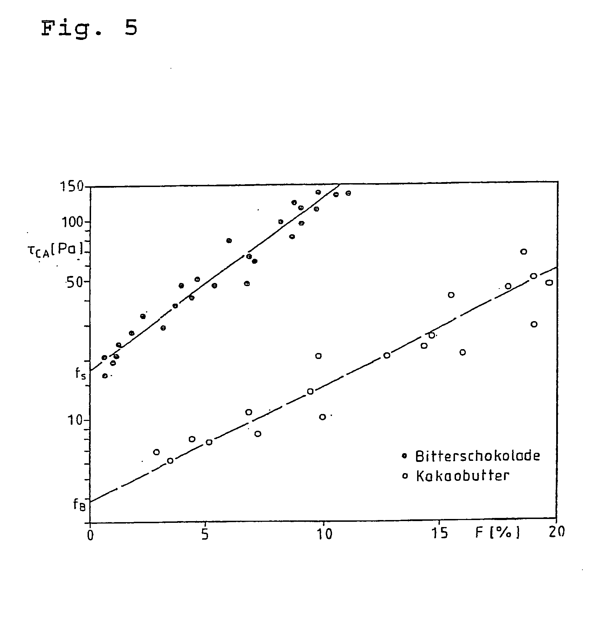

Flüssige Schokoladenmasse besitzt je nach Fett- und Kakaogehalt typischerweise eine dynamische Viskosität in einer Größenordnung von 1 bis 20 Pas. Zur Vorkristallisation durchläuft die Schokoladenschmelze eine Temperiermaschine, um unter Kühlung und Scherung erste Kristallkeime in der Fettphase zu erzeugen. Die danach vorkristallisierte (= temperierte) Schokoladenmasse ist nur im Temperaturbereich ca. 27 bis 33 °C stabil, bei tieferer Abkühlung kristallisiert sie spontan, bei weiterem Aufwärmen schmelzen die Kristallkeime. Typischerweise beträgt die dynamische Viskosität einer beispielhaften Schokoladenschmelze bei einer Temperatur von 34 °C ca. 10 Pas; die Fließgrenze liegt bei 20 Pa. Diese Temperierung ist für die weitere Verarbeitung und Ausformung der Schokoladenmasse notwendig. Vorkristallisierte Massen zeigen gegenüber der Schmelze ein stark verändertes Fließverhalten. So wurde gezeigt, dass vorkristallisierte Massen wesentlich höhere Fließgrenzen aufweisen (G. Ziegleder, M. Kegel, C. Santos, Fließverhalten vorkristallisierter Schokoladenmassen. ZSW 1990, 316-321; J. Kleinert, Rheologie der Schokolade. ZSW 28 (1975, 187-191). Rheologische Messungen an vorkristallisierten Massen in Laborgeräten sind wegen möglicher Temperaturunterschiede schwierig, da sie sich bei geringer Temperaturabweichung in ihrer entscheidenden Eigenschaft (Gehalt an Fettkristallen) verändern. Die Auswirkung des Gehalts an Fettkristallen auf die Fließgrenze ist in Fig. 5 anhand der Beispiele Bitterschokolade und Kakaobutter dargestellt. Man erkennt eine drastische Erhöhung der Fließgrenze mit zunehmendem Kristallgehalt. Auch verändern sich Massen beim Transport von der Produktionsanlage in ein Labor, da vorkristallisierte Massen (typische rheologische Messwerte bei einer Temperatur von 30 °C: dynamische Viskosität: 15 Pas, Fließgrenze: 50 Pa) bereits während einer kurzen isothermen Haltezeit ein ausgeprägt thixotropes Verhalten entwickeln (G. Ziegleder, M. Kegel, C. Santos, Fließverhalten vorkristallisierter Schokoladenmassen. ZSW 1990, 316-321), d.h. steigende Fließgrenzen entwickeln und höher viskos werden. Somit wird der Bedarf der Messung von Fließgrenze und Viskosität an vorkristallisierten bzw. temperierten Massen während der Produktion deutlich.

**Anwendungsbeispiele**

Tabelle 2 zeigt Fließgrenzen geschmolzener Schokoladen und Süßwarenmassen, die in einem modernen Rheometer gemessen bzw. nach Casson und OICCC berechnet wurden, und zum Vergleich Fließgrenzen, wie sie nach oben genannten Gleichungen aus der Resthaftmenge auf einem senkrechten Messkörper (bzw. schiefen Ebene) ermittelt wurden. Die relativ gute Korrelation zwischen OICCC-Daten und Messung anhand schiefer Ebene wird deutlich.

**Tabelle 1**

| Pas = kg/s·m | Dynamische Viskosität [Pas] | Fließgrenze [Pa] |
|---|---|---|
| Schokolade, geschmolzen | 0,1-20 | 0,1-30 |
| Kakaobutter, geschmolzen | < 1 | 0 |
| Schokolade, vorkristallisiert | 10-50 | 20-100 |
| Kakaobutter, vorkristallisiert | 0-5 | 1-40 |
| Milch (0-160 °C, 0-12 % Fett) | $4 \cdot 10^{-3}$ | -- |
| Pflanzenöl | $2\text{-}30 \cdot 10^{-3}$ $2\text{-}30 \cdot 10^{-3}$ | |
| Süßwarenmassen | -- | 10-100 |
| Tomatensaft (Trockensubstanz, Temp.) 0,01-0,4 : 10 % Trockensubstanz 0,7-4,3 : 25 % Trockensubstanz | | |
| Milch | | |
| Sahne | 0,8-1,5 | |
| Vollmilch | 18 | |
| Kondensmilch, gezuckert | 12,550 | |
| Buttermilch | 16 | |
| Molke | 16 | |
| Maische | 1-300 | |
| Wein | 0,501-0,502 | |
| Biscuitteig | 11 | |
| Karamelmasse | 18-950 | |
| Kakaomasse | 3-35 | |
| Sirup (20 °C) | 22-10,500 (je nach Trockensubstanz) | |
| Körperlotion | | |
| Hustensaft | | Keine Fließgrenze |
| Honig | 6-60 (je nach Feuchtigkeit) | |
| Blut | $4 \cdot 10^{-3}$ | |
| Glycerin | 10 | |
| Wasser (20 °C) | $1 \cdot 10^{-3}$ | |
| Benzol | $0,6 \cdot 10^{-3}$ | |
| Ricinusöl | 1,06 | |
| Melasse | 150 | |
| Glas | $> 10^{14}$ | |

**Tabelle 2**

| | Fließgrenze (Pa) bei 40 °C | | |
| --- | --- | --- | --- |
| | Rheometer extrapoliert, Doppelbestimmung | | Berechnet aus Resthaftmenge /schiefe Ebene |
| | OICCC/1 | OICCC/2 | |
| Milchschokolade | 19,85 | 20,38 | 21,1 |
| Bitterschokolade | 33,14 | 36,31 | 33,4 |
| Kakaobutter | 0 | 0 | nicht messbar |
| Füllmasse 1 | 43,34 | 44,64 | 42,0 |
| Füllmasse 2 | 49,81 | 49,04 | 47,9 |

**Patentansprüche**

1. Verfahren zum Messen der Fließgrenze von fließfähigen Stoffen in einem Strom des fließfähigen Stoffes **dadurch gekennzeichnet, dass** ein Messkörper iterativ von dem zu messenden fließfähigen Stoff bei gegebener, konstanter Temperatur benetzt wird, man anschließend den fließfähigen Stoff partiell für eine vorgegebene Ablaufzeit, die so bemessen ist, dass der Messkörper näherungsweise Gewichtskonstanz erreicht, ablaufen lässt und über die verbleibende Resthaftmenge am Messkörper die Fließgrenze bestimmt wird.

2. Verfahren nach Anspruch 1, **dadurch gekennzeichnet, dass** der Messkörper in den fließfähigen Stoff eingetaucht und herausgezogen wird oder dass der Messkörper von dem fließfähigen Stoff überspült wird.

3. Verfahren nach mindestens einem der vorangehenden Ansprüche, **dadurch gekennzeichnet, dass** die Zeit des Eintauchens bzw. Überspülens des Messkörpers in Abhängigkeit von der Viskosität des zu messenden fließfähigen Stoffes ausgewählt und so bemessen wird, dass eine vollständige Benetzung des Messkörpers und/oder ein vollständiger Austausch des fließfähigen Stoffes gewährleistet ist.

4. Verfahren nach mindestens einem der vorangehenden Ansprüche, **dadurch gekennzeichnet, dass** das Gewicht und/oder das Volumen der dem Messkörper nach der Ablaufzeit anhaftenden Resthaftmenge des fließfähigen Stoffes bestimmt wird, wobei bevorzugt die Bestimmung des Gewichts und/oder des Volumens über Sensoren, ausgewählt aus der Gruppe bestehend aus Waage, Kraftaufnehmer, Schwingungssensor, optische Sensoren, elektromagnetische Sensoren, piezoelektrische Sensoren und/oder Druckaufnehmer erfolgt.

5. Verfahren nach mindestens einem der vorhergehenden Ansprüche, **dadurch gekennzeichnet, dass** vertikale und/oder geneigte Flächen des Messkörpers benetzt werden.

6. Verfahren nach mindestens einem vorhergehenden Ansprüche, **dadurch gekennzeichnet, dass** das Verfahren so durchgeführt wird, dass die anhaftende Resthaftmenge bei der Messung die annähernd gleichen rheologischen Eigenschaften wie der fließfähige Stoff, dem sie entnommen wurde, aufweist.

7. Verfahren nach mindestens einem der vorangehenden Ansprüche, **dadurch gekennzeichnet, dass** zwischen zwei Messzyklen der Messkörper in dem fließfähigen Stoff temperiert und/oder gespült wird.

8. Verfahren nach mindestens einem der vorangehenden Ansprüche, **dadurch gekennzeichnet, dass** die Messdaten elektronisch weiterverarbeitet und/oder gespeichert werden.

9. Verfahren nach mindestens einem der vorangehenden Ansprüche, **dadurch gekennzeichnet, dass** als fließfähiger Stoff eine Dispersion gemessen wird, die bevorzugt ausgewählt ist aus der Gruppe bestehend aus viskosen Massen im Lebensmittel- oder Pharmabereich, Kosmetika, Dispersionsfarben und/oder keramischen Massen gemessen wird.

10. Verfahren nach vorangehendem Anspruch, **dadurch gekennzeichnet, dass** die Fließgrenze von viskosen Massen im Lebensmittel-, Kosmetik und/oder Pharmabereich, ausgewählt aus der Gruppe bestehend aus Schokoladenschmelzen, Joghurt, Ketchup, Brotaufstrich, Marmelade und/oder Honig bzw. Cremes, Salben, Lotionen, Lippenstift gemessen wird.

11. Verfahren nach vorhergehendem Anspruch, **dadurch gekennzeichnet, dass** zusätzlich der Temperiergrad von Schokolade berechnet wird, wobei bevorzugt die Messung der Fließgrenze mit einer Messung der Temperatur des fließfähigen Stoffes kombiniert wird.

12. Messvorrichtung zum periodischen Messen der Fließgrenze von fließfähigen Stoffen (2), mit einem Messkörper mit definierter Oberfläche, der mit einer funktionellen Einheit zur Bestimmung der am Messkörper (1) nach der Ablaufzeit verbleibenden, anhaftenden Resthaftmenge des fließfähigen Stoffes in Verbindung steht und einem Mittel (2, 3) zur Gewährleistung der periodischen Benetzung des Messkörpers mit der Dispersion.

13. Messvorrichtung nach Anspruch 12, **dadurch gekennzeichnet, dass** das zur Gewährleistung der periodischen Benetzung des Messkörpers enthaltene Mittel eine Hebevorrichtung (3) zur Absenkung und Anheben des Messkörpers in bzw. aus dem fließfähigen Stoff ist oder dass das zur Gewährleistung der periodischen Benetzung des Messkörpers enthaltene Mittel eine Vorrichtung zum Abzweigen einer Teilmenge des fließfähigen Stoffes und zur Umleitung der Teilmenge auf den Messkörper enthalten ist.

14. Messvorrichtung nach vorhergehendem Anspruch, **dadurch gekennzeichnet, dass** die Vorrichtung zum Abzweigen einer Teilmenge des fließfähigen Stoffes ein mechanischer Schieber (2), eine Klappe und/oder ein Ventil ist.

15. Messvorrichtung nach mindestens einem der Ansprüche 12 bis 14, **dadurch gekennzeichnet, dass** der Messkörper die Form eines Stabes, einer Platte, eines Trichters, eines Kegels und/oder einer Kugel besitzt und dass der Messkörper bevorzugt vertikale und/oder geneigte Flächen, die von dem fließfähigen Stoff benetzt werden, aufweist.

16. Messvorrichtung nach mindestens einem der Ansprüche 12 bis 25, **dadurch gekennzeichnet, dass** die funktionale Einheit zur Bestimmung der am Messkörper nach der Abtropfzeit verbleibenden, anhaftenden Resthaftmenge des fließfähigen Stoffes Sensoren zur Bestimmung des Gewichts und/oder des Volumens, ausgewählt ist aus der Gruppe bestehend aus Waage, Kraftaufnehmer, Schwingungssensor, optische Sensoren, elektromagnetische Sensoren, piezoelektrische Sensoren und/oder Druckaufnehmern.

17. Messvorrichtung nach mindestens einem der Ansprüche 12 bis 16, **dadurch gekennzeichnet, dass** weiterhin eine elektronische Auswertungseinheit zur Weiterverarbeitung und/oder Speicherung der Messdaten, vorzugsweise ein Computer vorgesehen ist.

18. Verwendung der Messvorrichtung nach mindestens einem der Ansprüche 12 bis 17 zur kontinuierlichen Überwachung von fließfähigen Stoffen in einer Produktionsanlage, insbesondere zur Bestimmung des Temperiergrades von Schokoladenmassen.

**Claims**

1. Method for measuring the yield point of flowable materials in a flow of the flowable material,
   ***characterised in that***
   a measuring body is wetted iteratively by the flowable material which is to be measured at a given, constant temperature, the flowable material is subsequently allowed to flow away partially for a prescribed run-off time which is calculated such that the measuring body reaches approximately a weight constant, and the yield point is determined via the remaining residual adhesion quantity on the measuring body.

2. Method according to claim 1, **characterised in that** the measuring body is immersed in the flowable material and withdrawn or **in that** the measuring body is rinsed over by the flowable material.

3. Method according to at least one of the preceding claims, **characterised in that** the time of immersion or rinsing over of the measuring body is selected as a function of the viscosity of the flowable material which is to be measured and is calculated such that complete wetting of the measuring body and/or complete exchange of the flowable material is ensured.

4. Method according to at least one of the preceding claims, **characterised in that** the weight and/or the volume of the residual adhesion quantity of the flowable material which is adhering to the measuring body after the run-off time is determined, preferably the determination of the weight and/or of the volume being effected via sensors, selected from the group consisting of scales, force transducer, oscillation sensor, optical sensors, electromagnetic sensors, piezoelectric sensors and/or pressure transducers.

5. Method according to at least one of the preceding claims, **characterised in that** vertical and/or inclined surfaces of the measuring body are wetted.

6. Method according to at least one of the preceding claims, **characterised in that** the method is implemented such that the adhering residual adhesion quantity has, during measurement, approximately the same rheological properties as the flowable material from which it has been removed.

7. Method according to at least one of the preceding claims, **characterised in that**, between two measuring cycles, the measuring body is tempered and/or rinsed in the flowable material.

8. Method according to at least one of the preceding claims, **characterised in that** the measuring data are further processed and/or stored electronically.

9. Method according to at least one of the preceding claims, **characterised in that**, as flowable material, a dispersion is measured which is preferably selected from the group consisting of viscous materials in the foodstuff or pharmaceutical sphere, cosmetics, dispersion pigments and/or ceramic materials.

10. Method according to the preceding claim, **characterised in that** the yield point of viscous materials is measured, in the foodstuff, cosmetic and/or pharmaceutical sphere, selected from the group consisting of chocolate melts, yoghurt, ketchup, spreads for bread, jam and/or honey or creams, ointments, lotions, lipstick.

11. Method according to the preceding claim, **characterised in that**, in addition, the tempering degree of chocolate is calculated, preferably the measurement of the yield point being combined with a measurement of the temperature of the flowable material.

12. Measuring device for periodic measurement of the yield point of flowable materials (9), having a measuring body with a defined surface which is in contact with a functional unit for determining the adhering residual adhesion quantity of the flowable material which remains on the measuring body (1) after the run-off time and having a means (2, 3) for ensuring periodic wetting of the measuring body with the dispersion.

13. Measuring device according to claim 12, **characterised in that** the means contained for ensuring periodic wetting of the measuring body is a lifting device (3) for lowering and lifting the measuring body into or out of the flowable material or **in that** the means contained for ensuring periodic wetting of the measuring body comprises a device for diverting a partial quantity of the flowable material and for diverting the partial quantity to the measuring body.

14. Measuring device according to the preceding claim, **characterised in that** the device for diverting a partial quantity of the flowable material is a mechanical slide (2), a flap and/or a valve.

15. Measuring device according to at least one of the claims 12 to 14, **characterised in that** the measuring body has the form of a rod, a plate, a funnel, a cone and/or a sphere, and **in that** the measuring body preferably has vertical and/or inclined surfaces which are wetted by the flowable material.

16. Measuring device according to at least one of the claims 12 to 15, **characterised in that** the functional unit for determining the adhering residual adhesion quantity of the flowable material which remains on the measuring body after the dripping time with sensors for determining the weight and/or the volume is selected from the group consisting of scales, force transducer, oscillation sensor, optical sensors, electromagnetic sensors, piezoelectric sensors and/or pressure transducers.

17. Measuring device according to at least one of the claims 12 to 16, **characterised in that** furthermore an electronic evaluation unit for further processing and/or storage of the measuring data, preferably a computer, is provided.

18. Use of the measuring device according to at least one of the claims 12 to 17 for continuous monitoring of flowable materials in a production plant, in particular for determining the tempering degree of chocolate materials.

**Revendications**

1. Procédé de mesure de la limite d'écoulement de matières fluides dans un flux d'une telle matière,
   **caractérisé en ce qu'**
   on mouille de façon itérative un corps de mesure avec la matière fluide à mesurer à une température constante prédéfinie, puis, on laisse s'écouler partiellement la matière fluide, pendant une durée prédéfinie qui est mesurée de sorte que le corps de mesure atteigne approximativement un poids constant, et on détermine la limite d'écoulement à partir de la quantité résiduelle de matière fluide adhérant sur le corps de mesure.

2. Procédé conforme à la revendication 1,
   **caractérisé en ce que**
   le corps de mesure est plongé dans la matière fluide et extrait de cette matière ou est rincé avec celle-ci.

3. Procédé conforme à au moins l'une des revendications précédentes,
   **caractérisé en ce que**
   la durée d'immersion ou de rinçage du corps de mesure est choisie en fonction de la viscosité de la matière fluide à mesurer, et est déterminée de façon à garantir un mouillage de la totalité du corps de mesure et/ou un remplacement de la totalité de la matière fluide.

4. Procédé conforme à au moins l'une des revendications précédentes,
   **caractérisé en ce que**
   le poids et/ou le volume de la quantité résiduelle de matière fluide adhérant au corps de mesure après la durée prédéfinie, est déterminé, cette détermination préférentiellement s'effectuant au moyen de détecteurs choisis dans le groupe constitué par des balances, transducteurs de forces, détecteurs d'oscillations, détecteurs optiques, détecteurs électromagnétiques, détecteurs piézo-électriques, et/ou transducteurs de pression.

5. Procédé conforme à au moins l'une des revendications précédentes,
   **caractérisé en ce qu'**
   on mouille des surfaces verticales et/ou inclinées du corps de mesure.

6. Procédé conforme à au moins l'une des revendications précédentes,
   **caractérisé en ce qu'**
   il est mis en oeuvre de sorte que la quantité résiduelle adhérant lors de la mesure, présente à peu près les mêmes propriétés rhéologiques que la matière fluide à partir de laquelle elle a été prélevée.

7. Procédé conforme à au moins l'une des revendications précédentes,
   **caractérisé en ce que**
   entre deux cycles de mesure, le corps de mesure est mis en équilibre de température et/ou rincé.

8. Procédé conforme à au moins l'une des revendications précédentes,
   **caractérisé en ce que**
   les données de mesure sont traitées électroniquement et/ou mises en mémoire.

9. Procédé conforme à au moins l'une des revendications précédentes,
   **caractérisé en ce qu'**
   en tant que matière fluide, on mesure une dispersion qui est de préférence choisie dans le groupe formé par des masses visqueuses dans le domaine alimentaire ou pharmaceutique, des produits cosmétiques, des matières colorantes en dispersion et/ou des masses céramiques.

10. Procédé conforme à la revendication précédente,
    **caractérisé en ce qu'**
    on mesure la limite d'écoulement de masses visqueuses dans le domaine alimentaire, cosmétique et/ou pharmaceutique, choisies dans le groupe formé par les chocolats fondus, les yaourts, les ketchups, les pâtes à tartiner, les confitures, et/ou le miel et/ou les crèmes, pâtes, lotions, ou bâtons à lèvres.

11. Procédé conforme à la revendication précédente,
    **caractérisé en ce qu'**
    on calcule en outre le degré d'équilibre de température de chocolats, la mesure de la limite d'écoulement étant de préférence combinée à une mesure de la température de la matière fluide.

12. Dispositif de mesure pour la mesure périodique de la limite d'écoulement de matières fluides (2), avec un corps de mesure ayant une surface définie reliée à une unité fonctionnelle pour déterminer la quantité résiduelle de la matière fluide adhérant sur le corps de mesure (1) après la durée d'écoulement, ainsi qu'un moyen (2, 3) pour garantir le mouillage périodique du corps de mesure par la dispersion.

13. Dispositif de mesure conforme à la revendication 12,
**caractérisé en ce que**
le moyen prévu pour garantir le mouillage périodique du corps de mesure, est un dispositif de levage (3) pour abaisser et lever le corps de mesure dans la matière fluide, ou hors de celle-ci, ou **en ce que** le moyen prévu pour garantir le mouillage périodique du corps de mesure, est un dispositif pour dériver une quantité partielle de la matière fluide et pour transférer cette quantité partielle sur le corps de mesure.

14. Dispositif de mesure conforme à la revendication précédente,
**caractérisé en ce que**
le dispositif pour dériver une quantité partielle de la matière fluide, est un tiroir mécanique (2), un volet et/ou une soupape.

15. Dispositif de mesure conforme à au moins l'une des revendications 12 à 14,
**caractérisé en ce que**
le corps de mesure présente la forme d'une barre, d'une plaque, d'un entonnoir, d'un cône et/ou d'une sphère, et **en ce que** le corps de mesure présente de préférence des surfaces verticales et/ou inclinées qui sont mouillées par la matière fluide.

16. Dispositif de mesure conforme à au moins l'une des revendications 12 à 15,
**caractérisé en ce que**
l'unité fonctionnelle pour déterminer la quantité résiduelle de la matière fluide adhérant sur le corps de mesure après la durée d'égouttage, comporte des détecteurs pour déterminer le poids et/ou le volume choisis dans le groupe formé par des balances, transducteurs de forces, capteurs d'oscillations, détecteurs optiques, détecteurs électromagnétiques, détecteurs piézo-électriques et/ou transducteurs de pression.

17. Dispositif de mesure conforme à au moins l'une des revendications 12 à 16,
**caractérisé en ce qu'**
il est en outre prévu une unité de traitement électronique pour le traitement ultérieur et/ou la mise en mémoire des données de mesure, de préférence un calculateur.

18. Utilisation du dispositif conforme à au moins l'une des revendications 12 à 17, pour le contrôle continu de matières fluides dans une unité de production, en particulier pour déterminer le taux d'équilibrage de température de masses de chocolat.

Fig. 1

Scherkraft $\tau^{0,5}$

Steigung = Viskosität $\eta^{0,5}$

Fließgrenze $\tau_0^{0,5}$

Berechnung nach Casson
$\tau^{0,5} = (\eta_C D)^{0,5} + \tau_C^{0,5}$

Schergeschwindigkeit $D^{0,5}$

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Die Fließeigenschaften von Schokolade. **J. CHEVALLEY.** S. T. Beckett: Moderne Schokoladentechnologie. Behrs Verlag, 1990, 211-231
- **H. D. TSCHEUSCHNER ; SCHOKOLADE, SÜßWAREN.** Rheologie der Lebensmittel. Behrs Verlag, 1993, 431-470
- **H. D. TSCHEUSCHNER.** Rheologische Eigenschaften von Schokoladenmasse und deren prozessrelevanten Bedeutung. *Zucker- u. Süßwarenwirtschaft ZSW,* 1993, 136-147
- **WINDHAB E. ; ROLFES L.** *Messung des Fließverhaltens von Schokoladenmassen ZSW,* 1991, 401
- Untersuchungsmethoden des Office International du Cacao et du Chocolat. Methode Blatt 10-D/1973. Viskosität von Schokolade - Bestimmung der Casson-Fließgrenze und der Casson Viskosität. Verlag Glättli
- Viscosity of cocoa and chocolate products. Analytical Method. International Confectionery Association, vol. 46, 1-17
- Structure-Rheology Relationships in chocolate processing. **WINDHAB E.** G. R. Ziegler: Confectionery Science. Dep Food Science, 1997, 104-126
- **COUSSOT P ; BOYER S.** Determination of yield stress fluid behaviour from inclined plane test. *Rheol. Acta,* 1995, vol. 34, 534-543
- **COUSSET P.** Avalanche behaviour in yield stress fluids. *Physical Review Letters,* 2002, vol. 88 (17), 175501-175504
- **J. KLEINERT.** Handbuch der Kakaoverarbeitung und Schokoladeherstellung. Behr's Verlag, 1997, 402ff
- **COUSSET P.** Avalanche behaviour in yield stress fluids. *Physical Review Letters,* 2002, vol. 88 (17), 175501-175504
- **W. BARTUSCH.** Der Einfluss von Rüttelstößen auf die Fließeigenschaften von Schokoladenmassen. *Fette Seifen Anstrichmittel,* 1961, vol. 63, 721-729
- **A. BEAURY.** Semesterarbeit Fraunhofer IVV/FH Weihenstephan. *Vorbereitung,* 2006
- **S. T. BECKETT.** The Science of chocolate. *RSC Paperbacks,* ISBN 0-85404-600-3, 112
- Bostwick Consistometer. CSC Scientific Company Inc, 2003
- **G. ZIEGLEDER ; M. KEGEL ; C. SANTOS.** Fließverhalten vorkristallisierter Schokoladenmassen. *ZSW,* 1990, 316-321
- **J. KLEINERT.** Rheologie der Schokolade. *ZSW,* 1975, vol. 28, 187-191